# EUROPEAN PATENT SPECIFICATION

(11) **EP 3 658 116 B1**
(45) Date of publication and mention of the grant of the patent: **30.03.2022**
(21) Application number: 18756294.7
(22) Date of filing: 26.07.2018
(51) Int. Cl.: A61K 9/00, A61K 9/127, A61K 47/69

(54) **ADRENALIN-CONTAINING DOUBLE LAYERED LIPID VESICLES FOR USE IN THE TREATMENT OF CARDIAC EMERGENCIES**
ADRENALINHALTIGE DOPPELSCHICHT-LIPIDVESIKEL ZUR VERWENDUNG BEI DER BEHANDLUNG VON NOTFÄLLEN IN DER KARDIOLOGIE
VÉSICULES LIPIDIQUES À DOUBLE COUCHE CONTENANT DE L'ADRÉNALINE DESTINÉES À ÊTRE UTILISÉES DANS LE TRAITEMENT D'URGENCES CARDIAQUES

(30) Priority: 28.07.2017 IT 201700086879
(43) Date of publication of application: 03.06.2020
(73) Proprietor: Università Degli Studi di Cagliari, 09124 Cagliari (IT)
(72) Inventor: XANTHOS, Theodoros, 13122 Athens (GR); MURGIA, Sergio, 09129 Cagliari (IT); MELI, Valeria, 31023 Resana (Treviso) (IT); MONDUZZI, Maura, 09028 Sestu (Cagliari) (IT); SINICO, Chiara, 09012 Capoterra (Cagliari) (IT); SCHLICH, Michele, 09126 Cagliari (IT); D'ALOJA, Ernesto, 09131 Cagliari (IT); LOCCI, Emanuela, 09131 Cagliari (IT)
(74) Representative: Primiceri, Maria Vittoria
(86) International application number: PCT/IB2018/055606
(87) International publication number: WO 2019/021240

(56) References cited:
- EP-A1- 0 211 647
- US-A1- 2005 096 570
- US-A1- 2017 095 489
- US-B1- 6 486 206
- FARRELL S ET AL: "Controlled release of liposomes", JOURNAL OF MEMBRANE SCIENCE, ELSEVIER BV, NL, vol. 127, no. 2, 14 May 1997 (1997-05-14), pages 223-227, XP004068506, ISSN: 0376-7388, DOI: 10.1016/S0376-7388(96)00284-0
- E. KISAK ET AL: "The Vesosome - A Multicompartment Drug Delivery Vehicle", CURRENT MEDICINAL CHEMISTRY, vol. 11, no. 2, 1 January 2004 (2004-01-01), pages 199-219, XP55090779, ISSN: 0929-8673, DOI: 10.2174/0929867043456197
- GRUBSTEIN B ET AL: "STABILIZATION OF EPINEPHRINE IN A LOCAL ANESTHETIC INJECTABLE SOLUTION USING REDUCED LEVELS OF SODIUM METABISULFITE AND EDTA", JOURNAL DRUG DEVELOPMENT AND INDUSTRIAL PHARMACY, NEW YORK, NY, US, vol. 18, no. 14, 1 January 1992 (1992-01-01), pages 1549-1566, XP002056303, ISSN: 0363-9045, DOI: 10.3109/03639049209040858

## Description

The present invention relates to double layered lipid vesicles, preferably liposomes, containing adrenaline incorporated inside them, for use in the treatment of cardiac emergencies.

The invention also relates to a pharmaceutical composition comprising said lipid vesicles, preferably in the form of an injectable liquid solution, in particular for parenteral administration during cardiac emergencies.

### PRIOR ART

Adrenaline (or epinephrine) is a hormone and a neurotransmitter belonging to the class of compounds called catecholamines, since their structure comprises both an *ortho*-dihydroxy benzene (whose chemical name is catechol) and an amine group.

Physiologically, it is secreted by chromaffin cells of the medullar region of the adrenal gland following stimulation by the sympathetic nervous system as a consequence of strong emotions, fear in particular, and in general in situations in which a "fight or flight" reaction may be necessary, i.e. where there may be the need for an escape, a fight, or in any event an increase in physical activity. Precisely for this reason, the effects of adrenaline at a systemic level include: gastrointestinal relaxation, a dilatation of the bronchi, an increase in heart rate and stroke volume (and consequently cardiac output), a diversion of blood flow towards muscles, the liver, the heart and the brain and an increase in glycaemia. The cell receptors that bind adrenaline and transmit the signal mediated by it are called adrenergic receptors.

Since the effects of adrenaline are varied and very intense, and involve numerous regions of the human body, the molecule has been used for some time as a drug in cardio-pulmonary resuscitation, typically by parenteral administration: its action is in fact fundamental in the event of a cardiac arrest, since it is capable of stimulating myocardial contraction and resumption of the heartbeat.

The reason for using adrenaline lies in its action on α-adrenergic receptors, which trigger arteriolar vasoconstriction in the periphery (fundamental for restarting spontaneous circulation) and vasoconstriction of the veins, with a consequent redistribution of blood to vital organs. Adrenaline thus diverts blood flow away from the periphery to central circulation, protecting vital organs from ischaemia through the redistribution of the volume. This action leads to an increase in the coronary perfusion pressure, which is a prognostic factor for the restoration of spontaneous circulation. Moreover, since adrenaline does not penetrate through the blood-brain barrier, the cerebral perfusion pressure increases indirectly, as a result of the volume redistribution. However, as it is a non-selective adrenergic receptor agonist, adrenaline exerts its action both on α receptors and β receptors, which have a differential distribution in the body and mediate different effects. The action of adrenaline on β1 receptors provokes vasodilation and activation of the β1 receptors of the heart, which mediate positive inotropic and chronotropic effects. Thus, adrenaline increases myocardial contraction, thereby increasing the oxygen demand of a heart already deficient in oxygen due to the cardiac arrest.

Furthermore, since α-adrenergic receptors are also expressed at the level of brain microvessels, a vasoconstriction occurs at the level of brain microcirculation, which exacerbates the ischaemia after the return of spontaneous circulation and increases the risk of neurological damage. Moreover, although the use of adrenaline favours the return of spontaneous circulation, it has been observed that in the event of a prolonged cardiac arrest, patients' likelihood of survival decreases with the repeated administration of adrenaline.

At present, the guidelines for cardio-pulmonary resuscitation provide for intravenous administration of a solution comprising adrenaline at a dose of 0.014 mg/kg of body weight (corresponding to 1 mg for an adult weighing 70 kg) every 3-5 minutes during cardio-pulmonary resuscitation. The frequency of administration is due to the brief half-life (and hence brief duration of action) of adrenaline *in vivo* (no greater than 5 minutes), a fact that makes repeated administration necessary in order to maintain the therapeutic concentration in the target tissue.

Repeated administrations exacerbate, in turn, the side effects of adrenaline, leading to a significant reduction both in the duration and quality of the life of patients, above all due to the neurologic deficits induced by the treatment.

The adrenaline solutions commonly available on the market and used in cardio-pulmonary resuscitation contain the active ingredient in concentrations of from 0.15 to 1 mg/ml, sodium chloride as an isotonifier, hydrochloric acid to bring the pH to 2.5 and permit the dissolution of the adrenaline, sodium metabisulphite to prevent oxidation and water for injectable preparations.

In the light of the above, therefore, it appears that the administration of adrenaline during cardio-pulmonary resuscitation can give rise to major side effects, the reason why the relationship between the risks and benefits of treatment with adrenaline is still a subject of debate.

In particular, high doses of adrenaline, useful for an immediate resuscitation effect, and high doses deriving from cumulative administrations increase the severity of neurologic side effects. For this reason, it appears to be of great importance to develop a system for the administration of adrenaline that may enable a controlled release in the body, reducing the number of administrations, the cumulative dose and, accordingly, the side effects.

Liposomes are vesicular structures that are substantially spherical in shape and made up of one or more double layers (bilayers) of phospholipids, whose composition is similar to that of cell membranes. Being biocompatible and nontoxic, liposomes have long been used in medicine as a drug delivery system, i.e. as vehicles for delivering molecules with a pharmacological or biological action into the body. In their simplest structure (unilamellar liposomes), liposomes are formed by a single bilayer of phospholipids; the phospholipids of the outer layer expose the polar heads towards the surrounding environment, whilst the nonpolar tails are turned towards the inside, where they interface with the nonpolar tails of the second lipid layer, whose organisation mirrors the previous one, i.e. the polar heads are turned towards the cavity of the liposome, which delimits an aqueous core. Unilamellar liposomes typically have a diameter comprised between 50 and 500 nm. Multilamellar liposomes, by contrast, are characterised by the presence of various concentric lipid bilayers, separated from one another by aqueous environments ("onion-like" structure). Multilamellar liposomes reach diameters comprised between 500 and 10000 nm.

Given that, as said, an aqueous environment (or more than one aqueous environment) comes to be created inside liposomes, liposomes can incorporate and deliver both hydrophilic molecules (in the aqueous core) and lipophilic molecules (inside the bilayer, between the nonpolar tails of the phospholipids) to the site of action. The preparation techniques and surface modifications enable the production of various liposomes for different applications. In fact, there are different methods for preparing liposomes with different characteristics in terms of size, lamellarity and encapsulation effectiveness. In particular, the encapsulation of drugs can be obtained by passive loading during liposome preparation, without any further steps, or with active loading processes, which usually result in greater efficiency compared to passive methods. Active loading (or remote loading) results in the drug's diffusion inside the empty liposome following the creation of a gradient between the inside and outside. For example, drugs can be encapsulated in response to the transmembrane pH gradient generated by acidic buffers present in the inner core or by dissociable proton-generating salts such as ammonium sulphate. Such procedures are known in the state of the art. For example, in order to generate a transmembrane pH gradient, liposomes can be hydrated with a known pH buffer, then dialysed in excess of another buffer with a different pH, to replace the buffer outside the liposomes. The external buffer is selected so that it will not impart any electrical charge to the molecule; the buffer present in the inner core of the liposome, by contrast, is selected so that it will electrically charge the molecule, once it has passed through the bilayer and entered the liposome. Since the charged molecules cannot diffuse through the lipid bilayer, the molecule remains trapped inside the liposome.

For example, WO2013114377 describes liposomes comprising an amphipathic base and internally loaded with an antitumour drug (doxorubicin, vincristine, topotecan), as well as a method for preparing them by means of an ammonium gradient.

EP361894 describes a method for preparing liposomes comprising an amphipathic drug in a pH gradient, which is formed by creating an ammonium gradient in this case as well.

WO2012118376 relates to a method for encapsulating in liposomes poorly water soluble substances, whose solubility was previously increased by association with a complexing agent or a co-solvent. The liposomes thus obtained are effective for delivering drugs to the central nervous system by enabling them to cross the blood-brain barrier.

Similarly, US20140220112 also describes a system for delivering molecules with low water solubility into the body by means of a formulation comprising a complex between such molecules and cyclodextrins and lipid vesicles internally loaded with such molecules, in a non-complexed form. The molecules that can be delivered with this system are, for example, antitumour or anti-inflammatory drugs.

US 6486206 (Lurie) teaches that epinephrine could be administered for resuscitating a patient from cardiac arrest. Lurie only mentions liposomes as suitable carriers, but nothing else is taught to solve the critical issues connected with the unstable nature of epinephrine and its loading into liposomes.

US 2017/095489 (Helson) suggest that a lipid mixture could help in the prevention or treatment of channelopathies or cardiac alterations. The fact that epinephrine appears in this list is not surprising and does not conflict with the present invention since in US 2017/095489 the lipid mixture act as a medication/preventive agent to treat a cardiac alteration which could be induced, among several other agents, by epinephrine. However, it is no mentioned whether the lipids have to be organized to form liposomes, and - even more important - the lipids in this case act as a medication per se, without any additional component.

US 2005/096570 (Palazzolo) teaches to use epinephrine in the form of an aqueous solution. The need for a controlled release system for adrenaline is never mentioned in US 2005/096570, nor the advantages of using a controlled release system over an immediate release formulation (eg. adrenaline solution) is discussed. Therefore, the skilled person would not consider the possibility of encapsulating adrenaline in liposomes or any other drug delivery system after reading this document.

The preparation method employed in Farrell S et al (Farrell S et al "Controlled release of liposomes" JOURNAL OF MEMBRANE SCIENCE, ELSEVIER BV, NL vol. 127, n0 2, 14 May 1997 pages 223-227) (thin film hydration + extrusion) would not allow the efficient encapsulation of adrenaline, which requires a pH-gradient method. In addition the liposomes described in Farrell are not PEGylated, and would not be suitable for prolonged circulation in the bloodstream.

In EP 0211647 (Allergan) liposomes are mentioned only as suitable formulations for delivery of epinephrine. Only one example describes production with epinephrine (example 17) and the production steps are different from those of the present invention (for instance in terms of pH, nor cholesterol or PEG-lipids or antioxidants are mentioned). Accordingly, the properties of liposomes obtained through this method are also different from what is described in the present invention (multilamellar as per EP0211647, unilamellar as per the invention).

Kisak et al., The Vesosome - A Multicompartment Drug Delivery Vehicle, CURRENT MEDICINAL CHEMISTRY, vol. 11, no, 2, 1 January 2004, pages 199-219, presents a delivery system based on small unilamellar liposomes encapsulated in a large multilamellar liposome for the prolonged release of actives. Such drug delivery system - the vesosome - could be loaded exploiting a pH gradient method, and the theory behind the process is described in the document. A model drug (prochlorperazine) is used to investigate the impact of different conditions (temperature and order of the steps in the process) on the loading efficiency.

The task of the present invention is to provide a form for the administration of adrenaline that allows the known drawbacks of the prior art described above to be overcome by means of a formulation that is safer and less harmful for the patients who need it.

The present invention also has the object of providing an effective method for loading adrenaline into double layered lipid vesicles (preferably liposomes) by means of a pH gradient process optimised and developed by the Applicant.

The Applicant has in fact observed that in the prior art no concrete examples or experimental data are reported which demonstrate the effective encapsulation of adrenaline in double layered lipid vesicles. The preparation of adrenaline-containing vesicles according to the known pH gradient techniques known in the art in fact have various drawbacks tied precisely to the characteristics of adrenaline.

The process of the present invention makes it possible to overcome such critical aspects, related to the poor stability of adrenaline, which furthermore has various problems of solubility as well as problems of oxidation/degradation that makes its effective encapsulation in double layered lipid vesicles difficult.

A further object of the invention is to develop a system for the gradual and constant release of adrenaline into the body, which makes it possible to avoid repeated administrations of the molecule within close intervals of time in order to keep the available therapeutic dose substantially constant. Within the scope of this object, therefore, it is intended to provide a system for administering adrenaline, in particular during emergency treatments such as cardio-pulmonary resuscitation, which avoids compromising or damaging coronary and/or cerebral blood circulation.

### SUMMARY OF THE INVENTION

The invention relates to double layered lipid vesicles, preferably liposomes, containing adrenaline incorporated inside them, for use in the treatment of cardiac emergencies.

The invention relates to a pharmaceutical composition comprising said lipid vesicles, preferably in the form of an injectable liquid solution, in particular for parenteral administration during cardiac emergencies.

The invention also relates to a process for preparing adrenaline-containing lipid vesicles.

### BRIEF DESCRIPTION OF THE FIGURES

Figure 1 shows cryo-TEM images of adrenaline-containing liposomes. The scale corresponds to 200 nm (A) or 100 nm (B).
Figure 2 shows the small-angle X-ray scattering (SAXS) profile of adrenaline-containing liposomes. I (a.u.) = intensity (arbitrary units); q = scattering vector.
Figure 3 (A) shows the mean diameter and polydispersity index (PDI) of the liposomes at different storage times expressed in days. Figure 3 (B) shows the pH and the Z potential of compositions comprising adrenaline-containing liposomes at different storage times expressed in days.
Figure 4 shows the results of an *in vitro* experiment on adrenaline released from the liposomes over time expressed in hours; the experiment was conducted using the modified dialysis method using Franz cells. The points represent the mean ± standard deviation of at least six experiments.
Figure 5 represents a comparison between the "sample and separate" method and the modified dialysis method for studying the release of adrenaline from the liposomes *in vitro.* The points represent the mean ± standard deviation of at least three experiments.
Figure 6 is a graph that illustrates the biocompatibility of a liposomal formulation of adrenaline (ADR liposomes) and of an adrenaline solution (ADR solution) at different concentrations vis-à-vis human endothelial cells: Viable cells (%); adrenaline concentration.

### DESCRIPTION OF THE INVENTION

In the context of the present invention, the term adrenaline (or epinephrine) means the hormone which, at a physiological level, is produced and secreted by chromaffin cells of the medullar region of the adrenal gland following stimulation by the sympathetic nervous system; the adrenaline encapsulated in the double layered lipid vesicles according to the present invention can be either of natural origin (extracted from humans or other vertebrates) or synthetic origin, prepared by chemical synthesis. "Adrenaline" and "epinephrine" are understood as synonyms and are thus used here interchangeably. Furthermore, "adrenaline" also refers to the salts, derivatives and/or precursors thereof, provided that they are physiologically acceptable and pharmacologically suitable for use in the treatment of cardiac emergencies, even where not expressly indicated. Moreover, in the context of the present invention, "adrenaline" indicates the (-) enantiomer, the (+) enantiomer or the racemic mixture indistinctly. The expression "double layered lipid vesicles" means structures of a size comprised between a few tens and a few hundreds of nanometers, formed by at least one lipid bilayer and enclosing one or more hydrophilic environments within them. In contrast, the one or more environments comprised within each bilayer are hydrophobic, being delimited by the lipids themselves.

The expression "adrenaline-containing vesicles (or liposomes)" means that the adrenaline is encapsulated within the internal aqueous cavity (or in the internal aqueous cavities) delimited by the one or more lipid bilayers.

The double layered lipid vesicles that are most common and most widely used in the medical field, above all as carriers for drugs or other molecules of biological or therapeutic importance, are liposomes. In one embodiment of the invention, therefore, the adrenaline-containing double layered lipid vesicles can preferably be liposomes. As is well known in the art, liposomes are formed by at least one bilayer of one or more phospholipids.

The lipid bilayer of the vesicles described here, preferably liposomes, can consist essentially of one or more saturated phospholipids; in another embodiment, the bilayer can consist essentially of one or more unsaturated phospholipids. More typically, however, the bilayer can consist essentially of a mixture of saturated phospholipids and unsaturated phospholipids; for example, the bilayer can be based on lecithin. As is well known in the art, "lecithin" is a general term that indicates a class of phospholipids present in the majority of animal and plant tissues: lecithin is thus a mixture of phospholipids of various types, which can be isolated from various natural sources or purchased commercially.

In a preferred embodiment, the one or more phospholipids forming the bilayer of the lipid vesicles can be selected from the group consisting of: dipalmitoylphosphatidylcholine (DPPC), distearoylphosphatidylethanolamine (DSPE), distearoylphosphatidylcholine (DSPC), dipalmitoylphosphatidylethanolamine (DPPE), dipalmitoylphosphorylglycerol (DPPG), distearoylphosphorylglycerol (DSPG), dipalmitoylphosphate (DPPA), distearoylphosphate (DSPA), dipalmitoylphosphatidylserine (DPPS), distearoylphosphatidylserine (DSPS), palmitoyl-stearoylphosphatidylcholine (PSPC), dioleoylphosphatidylethanolamine (DOPE), dioleoylphosphatidylcholine (DOPC), palmitoyl oleoylphosphatidylcholine (POPC), stearoyl-oleoylphosphatidylcholine (SOPC), and combinations thereof.

The lipid vesicles, preferably liposomes, can preferably also comprise cholesterol, which, being a nonpolar molecule, is located at the level of the nonpolar "tails" of the phospholipids, inside the phospholipid bilayer. In lipid vesicles, and in liposomes in particular, cholesterol plays a dual function: it reduces the permeability of the lipid bilayer to solutes and improves the packing of the lipids, thus rendering the structure of the vesicles homogeneous and cohesive.

Although lipid vesicles, and liposomes in particular, have a physiological physicochemical structure, which mimics the cell membrane, their half-life *in vivo,* following parenteral administration, is rather short, since they are captured by mononuclear cells and phagocytised. Another problem tied to the use of lipid vesicles and of liposomes in particular has revealed to be their brief retention of drugs following parenteral administration *in vivo.* In order to improve the duration of circulation *in vivo* and the drug retention capacity, it is possible to modulate the size of the lipid vesicles and liposomes in particular, and their physicochemical characteristics.

In one embodiment of the present invention, therefore, the lipid vesicles, preferably liposomes, can have a mean diameter preferably comprised between 50 and 500 nm, more preferably comprised between 50 and 200 nm, even more preferably comprised between 50 and 100 nm. Double layered lipid vesicles of small dimensions (in particular those having a diameter smaller than 100 nm) are in fact capable of escaping the reticuloendothelial system (RES) with greater efficacy than those of larger dimensions, a fact that prolongs the time they remain in the bloodstream after administration.

In another preferred embodiment, the double layered lipid vesicles, preferably liposomes, can be unilamellar, i.e. made up of only one lipid bilayer, preferably a phospholipid bilayer: unilamellar vesicles in fact have smaller dimensions than multilamellar ones, which typically fall in the range specified above.

Furthermore, another preferred embodiment of the invention envisages that the lipids forming the outer layer of the lipid bilayer can be modified by binding one or more hydrophilic polymers to them. Typically, one or more hydrophilic polymers can be bound to the polar heads of the phospholipids present in the outer layer of the vesicles, preferably liposomes. In other words, the vesicles can bear one or more hydrophilic polymers on their outer surface, typically bound to the polar heads of the phospholipids present on their outer layer.

The presence of hydrophilic polymers on the outer surface of the vesicles imparts to them a longer half-life following parenteral administration. In particular, the one or more hydrophilic polymers prevent the capture of the vesicles by the RES (or at least make it difficult), since they form a sort of outer layer that prevents plasma protein from binding to the vesicles themselves (or at least makes it difficult). The presence of the one or more hydrophilic polymers on the outer surface thus increases the stability of the vesicles, ensuring that they remain in the bloodstream longer following parenteral administration, typically by intravenous injection.

The one or more hydrophilic polymers can preferably be selected from the group consisting of: polyethylene glycol (PEG), polyoxazolines, polyvinylalcohols (PVA), polyglycerol, polyvinylpyrrolidone (PVP), poly-N-hydroxypropylmethacrylamide (polyHPMA), polyaminoacids and combinations thereof.

Furthermore, in a preferred embodiment, the percentage of lipids, preferably phospholipids, bearing a hydrophilic polymer bound to them can be comprised between 4% and 15% (molar) relative to the total lipids forming the vesicle.

More preferably, the hydrophilic polymer can be PEG, without limitations in terms of molecular weight and degree of polymerisation; the PEG can preferably have a molecular weight comprised between 2 and 20 KDa. The hydrophilic polymers can be bound to any lipid forming the outer layer of the vesicles which is chemically suited to this bond. Typically, the hydrophilic polymers can be bound to phospholipids, at the level of their polar head, or to the cholesterol, at the level of the hydroxyl group; the hydrophilic polymers can preferably be bound to distearoylphosphatidylethanolamine (DSPE); more preferably, the hydrophilic polymer bound to DSPE can be PEG.

The lipid vesicles according to the invention, preferably liposomes, can further have a negative surface charge, which is imparted to them by negatively charged lipids and/or derivatives thereof present in the outer lipid layer of the vesicles themselves. Therefore, the outer lipid layer of the vesicles can comprise one or more negatively charged lipids and/or derivatives thereof (anionic lipids). Anionic lipids that can be present in the vesicles of the invention can for example be selected from the group consisting of phosphoglycerides, phosphatidylserine, phosphatidylglycerol. Among negatively charged lipid derivatives, it is possible to mention, for example, phosphatidylethanolamine conjugated to a ligand, for example a hydrophilic polymer or a directing agent. Phosphatidylethanolamine is a zwitterionic molecule that is electrically neutral but carries a positive charge (localised at the level of the amine group) and a negative charge (localised at the level of the phosphate group). When phosphatidylethanolamine is conjugated to a binder, the amine group that engages in the bond is transformed into an amide group, thus losing the positive charge: conjugated phosphatidylethanolamine thus becomes a negatively charged lipid derivative. Advantageously, the molecule conjugated to phosphatidylethanolamine can be selected between a hydrophilic polymer, as previously described, and a directing agent, i.e. a molecule capable of interacting with a biological target towards which it is desired to direct the lipid vesicle. In addition to phosphatidylethanolamine, other zwitterionic lipids and phospholipids can be conjugated to a binder as described above.

The percentage of negatively charged lipids can preferably be comprised between 1% and 40% (molar) relative to the total lipids forming the vesicles, more preferably comprised between 5% and 30% (molar). The percentage range as regards the content of negatively charged lipids is necessarily very wide, since it depends on various elements, such as, for example, correct loading of drug, stability of the structure and toxicity, which are in turn influenced by various factors. In the formulations described in the examples, the content of anionic lipid content is equal to 21% (molar).

The negative surface charge is another characteristic that increases the efficacy of the vesicles for the use described here, as it ensures that they remain longer in the bloodstream following parenteral administration, typically by intravenous injection.

The double layered lipid vesicles, preferably liposomes, containing adrenaline, salts, derivatives or precursors thereof, have application in the medical field, in particular for the treatment of cardiac emergencies. The use of adrenaline is in fact recommended by numerous international guidelines and commonly employed in clinical practice to deal with situations in which the heartbeat is seriously compromised. The treatment of cardiac emergencies generally falls under the heading of "cardiac (or "cardio-pulmonary") resuscitation", since it aims to restart a heart muscle with no beat or a beat that is irregular (arrhythmic) to a point where it does enable a blood output such as to ensure normal circulation.

Cardiac arrest (CA) is a clinical situation characterised by ineffectiveness or absence of cardiac activity, which results in a cessation of blood circulation and breathing. The primary treatment for cardiac arrest is, as mentioned, cardio-pulmonary resuscitation (CPR), which should begin as soon as the cardiac arrest is detected or even suspected, in order to maximise the possibility of patient recovery. In the event of cardiac arrest, the heartbeat could be in conditions of ventricular fibrillation or pulseless ventricular tachycardia (collectively called "shockable" rhythms), or show characteristics of pulseless electric activity or asystole ("non-shockable" rhythms). CPR treatment of both groups of conditions includes chest compression and artificial ventilation, whereas in the event of uneven rhythms an electric shock (defibrillation) is necessary. The objective of CPR is to prevent or decrease the brain damage due to hypoxia by restoring a partial flow of oxygenated blood (usually less than 30% of the normal flow) through an external pumping action and to restore spontaneous circulation. The administration of vasoactive drugs at this stage aims to increase systemic vascular resistance in order to obtain a higher perfusion pressure in the myocardium and in the brain and possibly to facilitate the return of spontaneous circulation.

Therefore, the double layered lipid vesicles, and preferably the liposomes, containing adrenaline, or salts, derivatives or precursors thereof can preferably be used for the treatment of conditions selected from the group consisting of: cardiac arrest, ventricular fibrillation, pulseless ventricular tachycardia, pulseless cardiac electric activity, cardiac asystole and combinations thereof. In particular the vesicles, preferably liposomes, can be used for the treatment of cardiac arrest.

The introduction of adrenaline into the aqueous internal cavity of the vesicles is achieved via a pH gradient process developed by the Applicant. This process enables the encapsulation of pharmaceutically active substances (for example, weak anphipathic bases such as adrenaline) in the vesicles by exploiting the pH difference between the aqueous internal cavity of the vesicles (acidic pH typically comprised between 2 and 6) and the external aqueous solution (alkaline pH typically comprised between 8.5 and 10). Since adrenaline is a weak base, in its non-ionised form it is capable of passing through the lipid bilayer of the vesicles. Then, in the acidic environment of the aqueous internal cavity of the vesicles, the adrenaline molecules undergo a protonation, which prevents their further passage through the membrane and therefore their exit from the vesicle itself. The preparation of the adrenaline-containing vesicles according to the pH gradient technique known in the art has drawbacks tied to the scant stability and solubility of adrenaline. These critical aspects have been overcome with the process of encapsulation of the present invention, which ensures an efficient encapsulation of the adrenaline: by way of approximation, more than 75%, preferably about 90%, of the adrenaline present in the starting solution is encapsulated. The encapsulation efficiency is understood as the ratio between the drug trapped inside the liposomes x100 and the total drug.

The process according to the present invention enables the preparation of adrenaline-containing double layered lipid vesicles, preferably liposomes, by virtue of a suitable adaptation of the pH gradient technique made by the Applicant. The process comprises the steps of:
a) preparing a buffer solution with an acidic pH comprised between 2 and 6, preferably comprised between 2.4 and 3.0;
b) adding a solution containing lipids to the buffer solution and maintaining under stirring until a vesicular dispersion is formed;
c) sonicating the vesicular dispersion for a time comprised between 60 and 200 seconds, preferably between 100 and 150 seconds;
d) adding at least one antioxidant substance suitable for pharmaceutical use;
e) adding a basic solution to the vesicular dispersion until reaching a pH of about 9, preferably comprised between 8 and 9, even more preferably comprised between 8.7 and 9.0;
f) adding adrenaline and maintaining the dispersion under stirring for a time comprised between 5 and 15 hours, preferably between 10 and 13 hours, to enable the diffusion of the adrenaline into the lipid vesicles, wherein steps a) - f) are performed in the order described.

The buffer solution with an acidic pH is preferably obtained from citric acid and disodium hydrogen phosphate or from glycine and hydrochloric acid. In one embodiment the buffer solution is heated to a temperature comprised between 50 and 80°C, preferably between 60 and 70°C, prior to the addition of the solution containing the lipids.

The solution containing lipids is prepared by mixing the lipids in an organic solvent, selected, for example, from chloroform, dichloromethane, ethanol, methanol and diethyl ether.

The lipids are preferably as described above.

The antioxidant substance added after sonication is selected from: sodium metabisulphite, potassium metabisulphite, sodium sulphite, sodium thiosulphate, methionine, monothioglycerol, α-tocopherol, ascorbic acid, citric acid, butylhydroxyanisole (BHA), butylhydroxytoluene (BHT) and mixtures thereof.

The antioxidants are used in a nontoxic concentration, which is such as to effectively prevent oxidation of the adrenaline: this concentration varies according to the selected antioxidant. By way of example, the maximum concentration of BHT for intravenous administration is 0.005 mg/ml and the maximum concentration of monothioglycerol for intravenous administration is 10 mg/ml. In a preferred embodiment, the antioxidant can be sodium metabisulphite: the maximum concentration of sodium metabisulphite for intravenous administration is 10 mg/ml.

The presence of the antioxidant prevents the oxidation of the adrenaline subsequently added into the vesicular dispersion.

The basic solution that is added in order to obtain a basic pH is a solution of a strong base selected from NaOH, KOH or the like.

Steps a) - f) of the process of the present invention are performed in the order described.

For example, it is preferable to add at least one antioxidant substance suitable for pharmaceutical use to the starting vesicular dispersion (comprising the empty lipid vesicles) after the sonication and before step e) of adding the basic solution; in fact, if the at least one antioxidant substance was added after the basic solution, it could induce a substantial modification of the pH, consequently undermining the pH gradient necessary for an efficient loading of the adrenaline into the double layered lipid vesicles.

The adrenaline is preferably added in step f) after alkalinisation of the dispersion (step e)), in order to prevent the degradation of the adrenaline due to oxidation.

In a preferred embodiment of the invention, in step f) the adrenaline is added in an amount such as to obtain a lipids/adrenaline weight ratio of less than 15, preferably comprised between 10 and 15, even more preferably equal to about 14.6.

A weight ratio of less than 15 makes it possible to use a smaller amount of lipid excipient to obtain the same drug effectiveness.

Another aspect of the invention relates to a pharmaceutical composition comprising the adrenaline-containing double layered lipid vesicles as described here.

Said composition can have application in the medical field, in the treatment of cardiac emergencies of various types, such as, for example, cardiac arrest, ventricular fibrillation, pulseless ventricular tachycardia, pulseless cardiac electric activity, cardiac asystole.

The pharmaceutical composition of the invention can be formulated with suitable excipients known to a person skilled in the art. In one embodiment, the composition can be formulated in liquid form, preferably as an injectable solution for parenteral administration, more preferably for intravenous administration.

The adrenaline-containing double layered lipid vesicles for use according to the present invention and the pharmaceutical compositions that comprise them, conceived as described here, are susceptible of numerous modifications and variants, all falling within the scope of the inventive concept; furthermore, all of the details may be replaced by other equivalent elements whose correspondence is known to the person skilled in the art.

Furthermore, it is to be understood that the characteristics of the embodiments described with reference to one aspect of the invention are to be considered valid also in relation to other aspects of the invention, even if not explicitly repeated.

### EXAMPLES

### Materials and methods

### 1. Materials

Dipalmitoylphosphatidylcholine (DPPC) and distearoylphosphatidylethanolamine-polyethylene glycol-2000 (DSPE-PEG) were purchased from Lipoid (Ludwigshafen, Germany). The (-) adrenaline, cholesterol, sodium metabisulphite, citric acid monohydrate and disodium hydrogen phosphate dihydrate were purchased from Sigma-Aldrich (Milan, Italy). All the other products used are analytical grade.

### 2. Preparation of empty liposomes with acidic internal pH

A buffer solution at pH 2.60, containing citric acid monohydrate (0.094 M) and disodium hydrogen phosphate dihydrate (0.022 M) was prepared first of all. 5 ml of this solution was heated at 65 °C in a temperature-controlled bath. In a separate test tube the lipids: DPPC (27 mg), cholesterol (13 mg) and DSPE-PEG (10,2 mg) were mixed and dissolved in the minimum required amount of chloroform. The lipid solution obtained was poured slowly into the hot aqueous buffer solution under magnetic stirring, which was maintained until complete evaporation of the organic solvent and the formation of multilamellar vesicles of large dimensions. The vesicular dispersion was cooled to room temperature and sonicated with an immersion ultrasound apparatus with a titanium probe, for a total of 120 seconds divided into 5-second sonication cycles alternating with 2-second pauses (Soniprep 150, MSE Crowley, UK). After cooling, the sodium metabisulphite (0.053 M) was added to the liposomal dispersion.

### 3. Alkalinisation of the external aqueous medium and incorporation of adrenaline inside the vesicles

The aqueous medium containing the liposomal dispersion was titrated with the addition of a 2 M NaOH solution until reaching a pH value of 9.00 ± 0.1, whereas the aqueous environment inside the vesicles maintained an acidic pH value. The solid (-) adrenaline was added to this dispersion until a concentration of 0.6 mg/ml was obtained. The mixture was left overnight at room temperature under constant magnetic stirring to enable the diffusion of the adrenaline into the liposomes. The drug not incorporated in the vesicles, deposited on the bottom of the test tube, was removed from the formulation by centrifugation at 2000 rpm.

### 4. Physicochemical characterisation of the liposomes

The mean diameter and polydispersity index (PDI) of the liposomes were measured by means of the dynamic light scattering (DLS) technique, using a Zetasizer Nano device (Malvern Instruments, UK). The Zeta potential was measured with the same Zetasizer Nano device, with the M3-PALS (Phase Analysis Light Scattering) technique. All of the samples of the liposomal dispersions were analysed 1 hour after preparation or as indicated below.

The X-ray scattering measurements were recorded with an S3-MICRO SWAXS apparatus (HECUS X-ray Systems, Graz, Austria). The Cu Kα radiation with a wavelength of 1.542 Å was emitted by a GeniX X-ray generator, which operates at 50 kV and 1mA. A 1D-PSD-50 M system (HECUS X-ray Systems, Graz, Austria) with 1024 channels (amplitude 54.0 µm) was used to measure the X-ray scattering. The operating interval of q (Å⁻¹) was 0.003 ≤ q ≤ 0.600, with q = 4π-sin(θ)λ⁻¹, which represents the scattering wave vector. In order to perform the analysis, the liposomal dispersions were loaded into glass capillaries with thin walls (2 mm). The diffraction profiles were recorded for 3 h and analysed by means of GAP (Global Analysis Program) software.

The GAP software enables the SAXS diffractogram of the double layered aggregation structures of the phospholipids (vesicles and lamellar phases) to be interpolated. In particular, the thickness of the membrane (d_{B}) is defined as 2(z_{H}+ 2σ_{H}), where z_{H} and σ_{H}, obtained from the interpolation of the SAXS curves, respectively represent the distance of the head group of the phospholipid from the centre of the bilayer and the amplitude of the polar head. For the latter parameter, a fixed value of 3 Å was considered. The morphology of the liposomes was visualised with Cryo-TEM microscopy, using a JEOL JEM-1230 Electron Microscope (Joel, Japan). The liposomal dispersion was loaded onto a Lacey Formvar/Carbon support, housed on a 300 mesh copper grid; the thin aqueous film was dried with filter paper and immediately placed in liquid ethane, using a Vitrobot apparatus (FEI, Hillsboro, OR, USA). The frozen grids were maintained in liquid nitrogen and transferred, again in liquid nitrogen, to a specific support (Gatan, Pleasanton, CA, USA) at about -180 °C. The images were recorded with a CCD camera (Pleasanton, CA, USA), at a voltage of 100 kV.

### 5. Encapsulation efficiency

The liposomal dispersions were purified by ultrafiltration to remove the non-incorporated drug using vertical filtering units with a cutoff of 30 kDa (Amicon Ultra 4, Merck Millipore). 3 ml of the liposomal dispersion was loaded into the upper compartment of the filtering unit and made to rotate at 4000 rpm for two 15-minute cycles, followed by two 30-minute cycles. After each cycle, the filtrate was removed from the lower compartment and an equivalent volume of citrate-phosphate buffer at pH 9 containing sodium metabisulphite (0.053 M) was added into the upper compartment.

The encapsulation efficiency (E%) was calculated by analysing, after the destruction of the vesicles, the concentration of drug present in the purified dispersions and in the non-purified ones by means of high-performance liquid chromatography (HPLC).

### 6. Drug release in vitro

The *in vitro* studies on the release of adrenaline from the liposomal formulations were conducted with a modified dialysis technique or by means of a "sample and separate" method.

In the former case, a cellulose membrane (Spectra/Por^{®}: cut-off 12-14 kDa MW, pore size 3 nm, Spectrum Laboratories Inc., USA) was positioned between the two compartments of a Franz vertical diffusion cell (Rofarma, Milan). The receptor compartment, with a capacity of 6.5 ml and a diffusion area of 0.636 cm², was filled with a phosphate buffer solution (PBS pH 7.4) containing sodium metabisulphite (0.053 M), stirred constantly by a small magnetic stirrer at a controlled temperature of 37 °C for the whole duration of the experiment. At the beginning of the experiment, 0.5 ml of liposomal dispersion and a solution of adrenaline in a citrate-phosphate buffer supplemented with sodium metabisulphite (0.053 M), titrated to pH 9 with NaOH 2 M, were applied on the surface of the membrane as a control.

At 1-hour time intervals, for the 8 hours of the experiment, 1 ml of the solution in the receptor compartment was removed and restored with fresh buffer. The amount of adrenaline contained in the samples removed was analysed by HPLC.

In the "sample and separate" method, 100 µl of liposomes was mixed with 10 ml of release medium in a glass test tube and kept under stirring at 37 °C. At regular time intervals (0.5 h, 1 h, 2 h, 4 h, 6 h, 8 h), 500 µl of the mixture was removed and centrifuged in an ultrafiltration unit (MWCO 10 KDa) to separate the free drug from the drug encapsulated in the liposomes. The amount of adrenaline contained in the samples removed was analysed by HPLC.

### 7. HPLC determination of the adrenaline

The adrenaline contained in the liposomes and in the release medium was determined by fluorescence spectroscopy at an excitation wavelength of 280 nm and emission wavelength of 310 nm, using an Alliance 2690 chromatograph (Waters, Italy). Use was made of an XTerra C18 column (3.5 µm, 4.6 x 150 mm, Waters), with a mobile phase composed of 86.5% water, 13.45% acetonitrile and 0.05% acetic acid (v/v) at a flow rate of 0.8 ml/min. A calibration curve was constructed using standard solutions (100 - 1 ng/µl), having a correlation coefficient (R²) of 0.999.

### 8. Biocompatibility

The biocompatibility of the formulation was tested on human umbilical vein endothelial cells (HUVECs) obtained from a pool of healthy donors (Promocell, Germany). The cells were cultured in Endothelial Cell Growth Medium 2 (ECGM 2, Promocell) enriched with penicillin (50 units/ml) and streptomycin (50 mg/ml), and maintained in an incubator with a humidified atmosphere, CO₂ (5 %) at 37 °C. The cells were seeded in 96-well plates 24 hours before the treatment with liposomes (density 4 x 104 cells/well). The adrenaline-containing liposomes or an adrenaline solution (used as a control) were mixed with the culture medium to obtain different concentrations of adrenaline (in the range of 3-120 µg/ml), and the cells were treated with the formulations for 5 hours. At the end of the treatment, the medium was removed and replaced with 100 µl of a solution of 3-(4,5-dimethylthiazol-2-yl)-2,5-diphenyltetrazolium bromide (MTT) in PBS (0.5 mg/ml). After 4 hours of incubation at 37 °C, the MTT was removed and 100 µl of dimethyl sulphoxide (DMSO) was added in each well. Absorbance was measured at 570 nm by means of a Synergy 4 plate reader (BioTek, Winooski, VT, USA). The results are expressed as a percentage of viable cells compared to the untreated cells.

### 9. Statistical analysis

The data analysis was performed with R software, version 2.10.1. The results are expressed as the mean ± standard deviation. The Tukey test was used to verify any statistical differences between groups, whilst the Student T test was used to compare two samples. Significance was tested at the 0.05 probability level (p).

### 10. In vivo study on animals

The study was conducted following authorisation of the project (Ref. No. 4263 2/8/2017) and in accordance with national provisions (PD 56/2013, as per Directive 2010/63/EU).

For the pilot study, three animals per group were used. The pig (Landrace/Large White sow, 20 kg, from the Validakis farm, Koropi, Greece) was selected as the model animal. The experimental protocol includes a comparison between 2 groups of animals: group A, receiving commercial adrenaline (adrenaline in an injectable solution 1mg/ml, Demo, Greece), and group B, receiving a formulation of adrenaline contained in liposomes (experimentally produced according to the present invention), both at a dose of 0.01 mg/Kg.

The measurements, conducted at different time intervals, include haemodynamic recordings: ECG, MAP, CVP, CO, SVR, collection of arterial blood samples for the measurement of pH, pO₂, pCO₂, base deficit, haemoglobin, lactate and collection of venous blood samples for the measurement of adrenaline and serum biochemistry and plasma metabolomics.

### Results

### Preparation of the liposomes

The adrenaline-containing liposomes were prepared exploiting a method of active loading, based on the difference in pH between the aqueous core of the liposomes and the external medium as the driving force for encapsulation. This technique is known in the literature for its effectiveness in loading weak bases or weak acids into liposomes with a higher encapsulation efficiency compared to passive loading methods (for example hydration of the lipid film). In order to induce the encapsulation of a weak base like adrenaline, the pH in the aqueous core of the liposomes must be acidic, whereas the pH of the external phase should be neutral or slightly alkaline. In fact, when adrenaline (pKa 8.6) is incubated at pH 9.00 in the presence of liposomes with an internal pH of 2.60, its neutral form can passively diffuse through the lipid bilayer to reach the aqueous core, where it is trapped in the protonate form, which is no longer able to pass through the membrane in the opposite direction.

One of the main limits of this loading technique is the stability of the drug in the pH range used. Adrenaline oxidises easily at alkaline pH levels, which promote the formation of the toxic derivative adrenochrome, which can be easily recognised because of the reddish brown colour it imparts to aqueous solutions. In order to prevent this phenomenon, the formulation includes an antioxidant, sodium metabisulphite, used at a concentration lower than the maximum concentration accepted by the Food and Drug Administration (FDA) for parenteral preparations for human use. The concentration of 10 mg/ml has proven to be sufficient to prevent adrenaline oxidation during the loading process and, together with the protection provided by encapsulation in the vesicles, has made it possible to obtain a long-term stability of the preparation, which shows no colouring for up to 15 months after preparation when stored at room temperature.

### Physicochemical characterisation of the liposomes

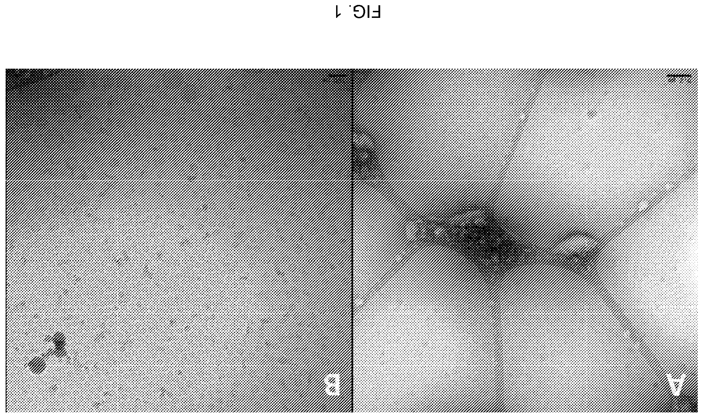

The morphology of the liposomes was initially evaluated by cryo transmission electronic microscopy (cryo-TEM). As may be seen from figures 1 A and B, the adrenaline-containing liposome formulation is a homogeneous dispersion of unilamellar vesicles. The inspection of the samples at a greater magnification reveals the presence of a small percentage of bilamellar liposomes, whereas higher levels of lamellarity were not observed. Certain sectors visible in figure 1 show an apparently regular packing of the liposomes, and this fact warrants a few comments. In fact, rather than being caused by specific interactions between the vesicles, this particular arrangement most likely originated from the drying procedure during the preparation of the sample for the microscopy experiments; this caused a thinning of the aqueous film at the centre of the carbon grid, where the smaller liposomes gathered, taking on an orderly appearance, since the majority of them had the same dimensions.

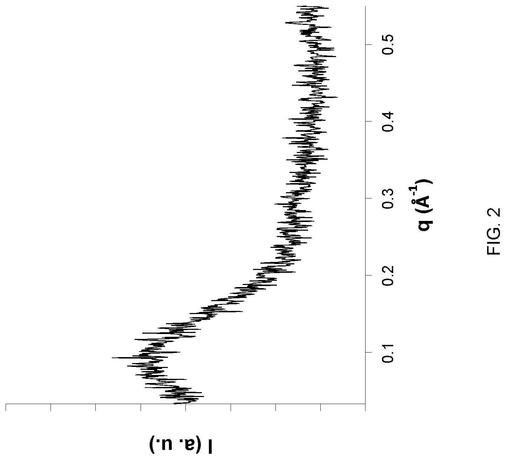

The formulation in question was then characterised by SAXS. The diffusion profile (figure 2) showed no Bragg (or quasi-Bragg) peaks, thus supporting the previously mentioned fact that the bilamellar liposomes represent a minimal percentage of the sample. Therefore, the SAXS profile was successfully interpolated by the GAP by exploiting a purely diffusive model. The best interpolation parameters obtained from this modelling were z_{H} = 19.0 Å and σ_{H} = 3.0 Å, which resulted in a bilayer thickness (dB) of 50 Å, a value in perfect agreement with others reported for similar systems.

The size, Zeta potential and polydispersity index (PDI) of the vesicles were measured by Dynamic Light Scattering. The results of these analyses show that the formulation fundamentally contains liposomes characterised by a low polydispersity index (lower than 0.2), with a mean diameter of 70 nm and a Zeta potential of about -6 mV.

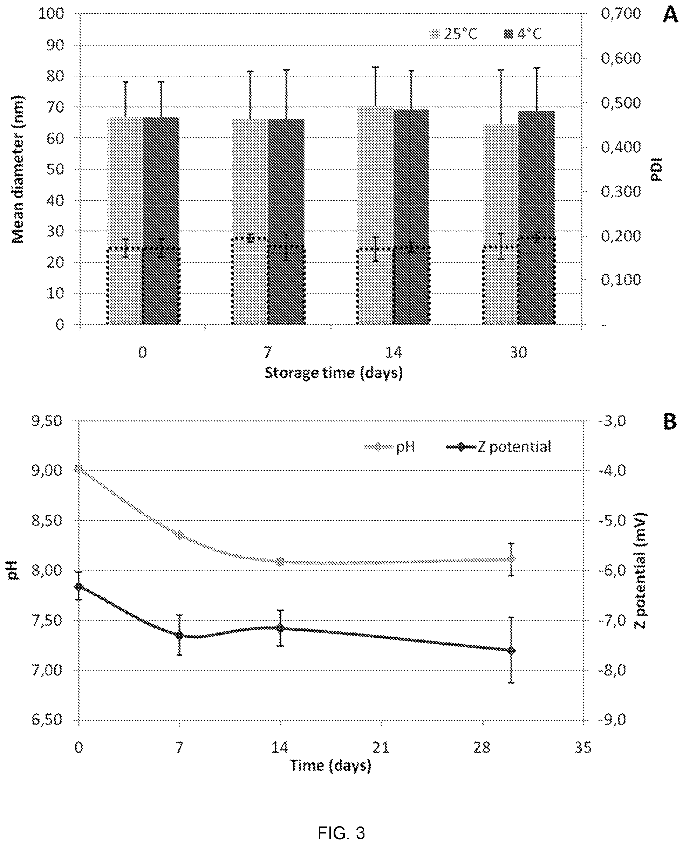

These parameters, together with the pH of the preparation, were monitored for 30 days in order to verify the stability of the system. As may be seen from figures 3 A and B, the size, PI and Z potential did not undergo any significant changes during storage, whilst during the first 14 days a decrease in pH of about 1 unit was observed, probably because of an initial redistribution of protons between the strongly acidic core of the liposomes (pH 2.60) and the relatively basic bulk phase (pH 9.00). Furthermore, the storage temperature (4 °C or 25 °C) does not seem to have an effect on the characteristics of the preparation.

### Encapsulation efficiency

The adrenaline-containing liposomes were prepared in lots composed of 3 samples each. In order to verify the repeatability of the production process, the encapsulation efficiency of every sample of each lot was analysed. The mean encapsulation efficiency for each lot was 89 ± 0.5 %, 78 ± 2.7 %, 87 ± 2.2 %. The standard deviation among the samples of the same lot was always lower than 3 %, which is considered an acceptable value for a system of this complexity.

### In vitro release studies

A necessary introductory note to this section is that there does not exist any standard method approved by a regulatory agency for conducting *in vitro* studies on the release from nanoparticle-based formulations. All of the methods used are derived in some way from release studies on conventional pharmaceutical forms (for example capsules, tablets, etc ...). The most significant results are the ones capable of establishing an A level *in vitro-in vivo* (IVIVC) correlation (linear or non-linear point-to-point correlation between absorption *in vivo* and release *in vitro*).

In order to best describe the kinetics of the release of adrenaline from the liposomes, the formulations were tested using two different *in vitro* release methods, and in particular a modified dialysis method using Franz cells and the "sample and separate" method.

In both cases use was made of PBS pH 7.4 at a controlled temperature of 37 °C to simulate the pH, osmolarity and temperature of plasma.

Initially, the modified dialysis method with Franz cells was used. Adrenaline release from the liposomes was evaluated one day, one week or one month after preparation to identify any differences deriving from destabilisation of the liposome membrane over time.

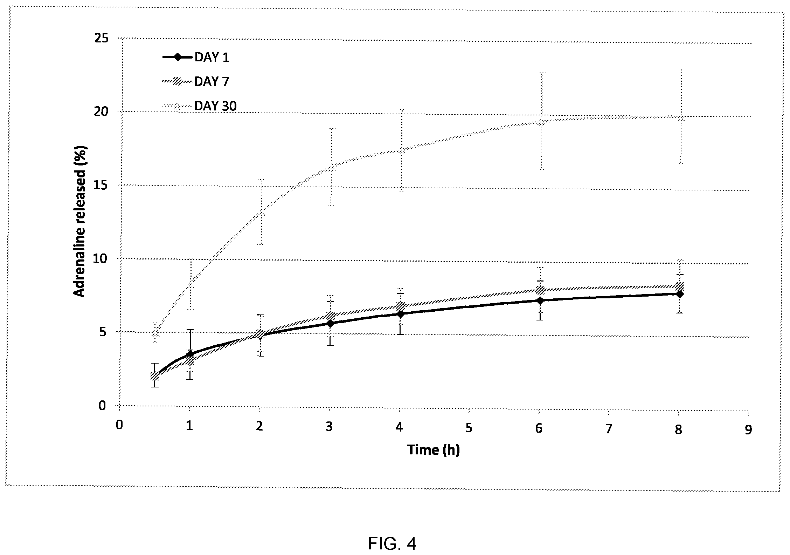

As may be seen from figure 4, the freshly prepared formulation showed a controlled release over time, with a maximum amount of adrenaline diffused from the vesicles equal to 8 ± 1 % of the dose used. A controlled release can also be noted after storage at room temperature, even though a higher release frequency was observed one month after the preparation of the liposomes (20 ± 3 % of the dose released after the 8 hours of the experiment). Indeed, since all three curves show a similar trend, though at different heights, we have hypothesised that the permeability of the bilayer may vary during prolonged storage as a consequence of the proton redistribution mentioned above and manifested through the lowering of the pH. In fact, the absence of changes in the mean diameter and in the Zeta potential during storage enables us to rule out a deterioration of the vesicular structure. On the other hand, no significant differences were observed in the release trend 7 days after preparation. The release from an adrenaline solution under the same conditions was monitored and used as a control, generating a total amount of drug equal to 70 ± 3 % after the 8 hours of the experiment.

The main limit of the modified dialysis method lies in the presence of the dialysis membrane which separates the liposomes from the receiving buffer solution. In fact, Franz cells offer a two-dimensional interface for the diffusion of the free drug from the formulation to the receiving solution. Therefore, although it is an excellent method for evaluating any differences between formulations, we judged this experimental setup to be unsuitable for representing the release that takes place in the circulatory system *in vivo.*

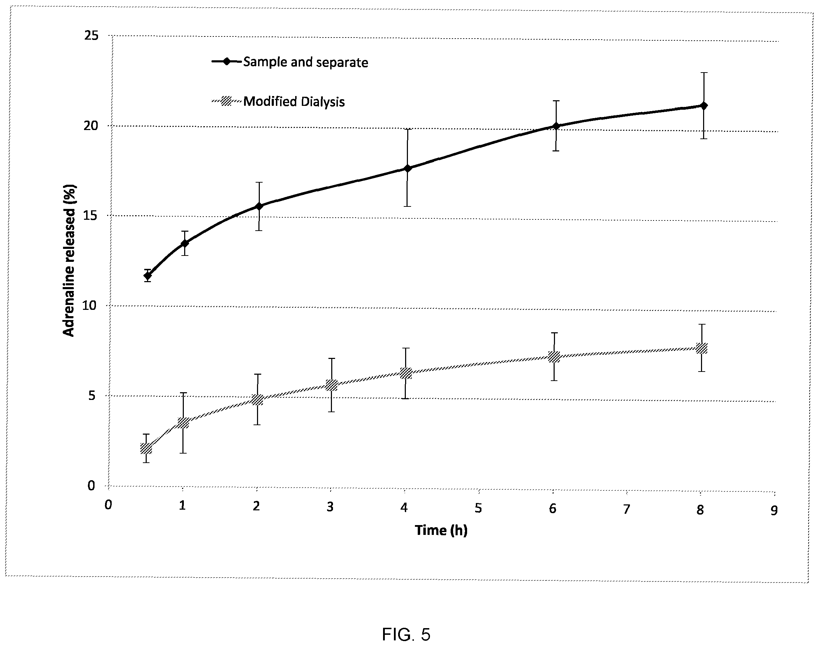

In contrast, in the "sample and separate" method the vesicles are placed in direct contact (without the interposition of filters or membranes) with the receiving solution, providing an environment that is more similar to circulation *in vivo.* The results obtained with this method are shown in figure 5, which also shows the results of the modified dialysis method (relating to the release experiment one day after preparation of the samples). One may observe an immediate release of about 11 % of the dose, a value in agreement with the amount of adrenaline not contained in the liposomes as revealed by the encapsulation efficiency experiments. In fact, the *in vitro* release studies were conducted using liposomal formulations that were not purified from the free adrenaline. Subsequently, the release kinetics appear to be relatively slow, leading to a maximum release of 23 % of the dose after the 8 hours of the experiment. Notwithstanding the significant difference in the total amount of adrenaline released, ascribable to the substantial difference between the two methods, both experimental approaches describe a prolonged release by the liposomes when they are exposed to simulated physiological conditions.

In this regard, according to need, the adrenaline release properties of the liposomes can be modified by varying the composition in terms of phospholipids, or including others that can stabilise or destabilise the vesicular structure at the physiological temperature and pH, thus leading respectively to a slower or faster release.

### Biocompatibility

Although all the components of the formulation prepared had been individually approved for clinical use in humans by one or more regulatory bodies, and therefore recognised as nontoxic at the concentrations used in the invention, the biocompatibility of the liposomal system described was tested on human umbilical vein endothelial cells (HUVEC). Cells of an endothelial type were selected with the objective of testing the effects of the composition on the tissue that is mostly exposed to it following intravenous injection.

Furthermore, HUVECs are non-cancer cells derived from healthy donors and therefore highly representative of the type of cells that come into contact with the adrenaline-containing liposomes when administered during a cardiac arrest.

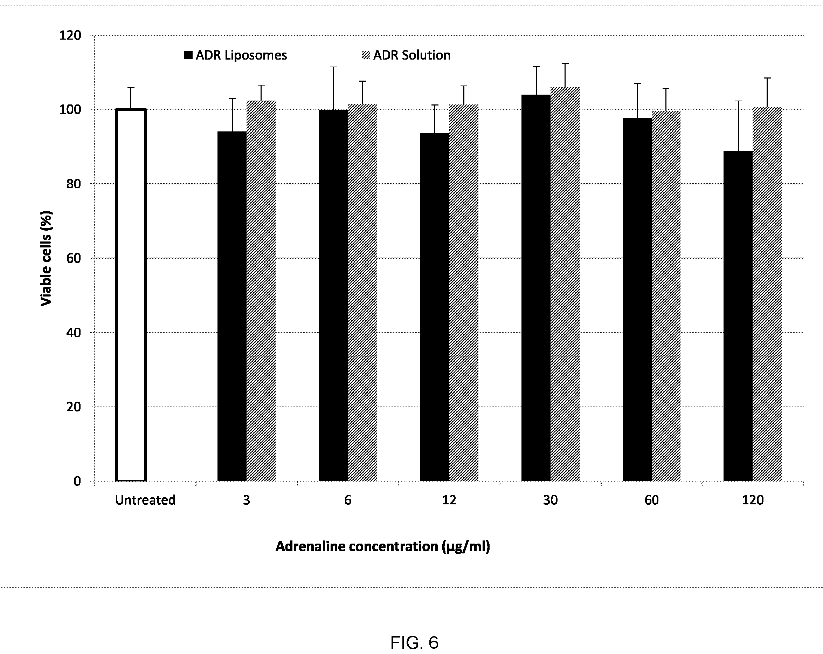

The cells were treated with liposomes or with an adrenaline solution, administered at different concentrations. As shown in figure 6, no significant difference was identified between the viability of treated and untreated cells, which demonstrates the biocompatibility of the composition when administered at a concentration of adrenaline of up to 120 µg/ml.

### In vivo study on animals

The aim of the experimental protocol followed was to study the adrenaline-containing liposome formulation of the present invention in comparison with a commercial formulation of adrenaline commonly used in cardio-pulmonary resuscitation. The experiments showed that the liposomes of the present invention do not produce any side effect. The 3 animals that received the liposomal formulation showed an immediate increase in heart rate and in systolic and diastolic pressures, but there was also an observed tendency (though not statistically significant at present) of those pigs to maintain their haemodynamic profile for a longer period of time compared to the animals who received the commercial formulation. This tendency is in agreement with the *in vitro* release studies, which show a controlled transfer of adrenaline from the liposomes over time, and represents a confirmation of the functioning of the system of the present invention.

## Claims

1. A process for preparing adrenaline-containing double layered lipid vesicles, preferably liposomes, comprising the steps of:
a) preparing a buffer solution with an acidic pH comprised between 2 and 6, preferably comprised between 2.4 and 3.0;
b) adding a solution containing lipids to the buffer solution and maintaining under stirring until a vesicular dispersion is formed;
c) sonicating the vesicular dispersion for a time comprised between 60 and 200 seconds, preferably between 100 and 150 seconds;
d) adding at least one pharmaceutically acceptable antioxidant substance;
e) adding a basic solution to the vesicular dispersion until reaching a pH of about 9, preferably comprised between 8 and 9, even more preferably comprised between 8.7 and 9.0;
f) adding adrenaline and maintaining the dispersion under stirring for a time comprised between 5 and 15 hours, preferably between 10 and 13 hours;
wherein steps a) - f) are performed in the order described.

2. The process according to claim 1, wherein the adrenaline is added in an amount such as to obtain a lipids/adrenaline weight ratio of less than 15, preferably comprised between 10 and 1 5, even more preferably equal to about 14.6.

3. The process according to claims 1-2, wherein the at least one antioxidant substance is selected from sodium metabisulphite, potassium metabisulphite, sodium sulphite, sodium thiosulphate, methionine, monothioglycerol, α-tocopherol, ascorbic acid, citric acid, butylhydroxyanisole (BHA), butylhydroxytoluene (BHT) and mixtures thereof, preferably sodium metabisulphite.

4. Adrenaline-containing double layered lipid vesicles, for use in the treatment of cardiac emergencies obtainable by the process according to any one of claims 1-3, wherein said double layer consists essentially:
i) of one or more saturated phospholipids, or one or more unsaturated phospholipids, or a mixture of one or more saturated phospholipids and one or more unsaturated phospholipids, and
ii) of one or more negatively charged lipids,
and wherein the lipids which form the outer layer of the lipid double layer are bound to one or more hydrophilic polymers and the double layered lipid vesicles comprise at least one pharmaceutically acceptable antioxidant substance, said vesicles containing more than 75%, preferably about 90%, of the adrenaline added in step f).

5. The lipid vesicles for use according to claim 4, wherein said lipid vesicles are liposomes.

6. The lipid vesicles for use according to any one of claims 4-5, wherein said one or more phospholipids are selected from the group consisting of: dipalmitoylphosphatidylcholine (DPPC), distearoylphosphatidylethanolamine (DSPE), distearoylphosphatidylcholine (DSPC), dipalmitoylphosphatidylethanolamine (DPPE), dipalmitoylphosphorylglycerol (DPPG), distearoylphosphorylglycerol (DSPG), dipalmitoylphosphate (DPPA), distearoylphosphate (DSPA), dipalmitoylphosphatidylserine (DPPS), distearoylphosphatidylserine (DSPS), palmitoyl-stearoylphosphatidylcholine (PSPC), dioleoylphosphatidylethanolamine (DOPE), dioleoylphosphatidylcholine (DOPC), palmitoyl oleoylphosphatidylcholine (POPC), stearoyl- oleoylphosphatidylcholine (SOPC) and combinations thereof.

7. The lipid vesicles for use according to any one of claims 4-6, wherein said one or more negatively charged lipids are selected from the group consisting of phosphoglycerides, phosphatidylserine, phosphatidylglycerol, phosphatidylethanolamine conjugated to a ligand, and combinations thereof.

8. The lipid vesicles for use according to any one of claims 4-7, wherein said one or more hydrophilic polymers are selected from the group consisting of: polyethylene glycol (PEG), polyoxazolines, polyvinylalcohols (PVA), polyglycerol, polyvinylpyrrolidone (PVP), poly-N-hydroxypropylmethacrylamide (polyHPMA), polyaminoacids and combinations thereof.

9. The lipid vesicles for use according to claim 8, wherein the hydrophilic polymer is PEG.

10. The lipid vesicles for use according to any one of claims 4-9, wherein said double layer comprises cholesterol.

11. The lipid vesicles for use according to any one of claims 4-10, wherein said vesicles have a mean diameter comprised between 50 and 500 nm, more preferably comprised between 50 and 200 nm, even more preferably comprised between 50 and 100 nm.

12. The lipid vesicles for use according to any one of claims 4-11, wherein said vesicles are unilamellar.

13. The lipid vesicles for use according to any one of claims 4-12, wherein said use is in the treatment of one or more conditions selected from the group consisting of: cardiac arrest, ventricular fibrillation, pulseless ventricular tachycardia, pulseless cardiac electric activity, cardiac asystole and combinations thereof.

14. A pharmaceutical composition comprising the lipid vesicles for use according to any of claims 4-13.

15. The pharmaceutical composition for use according to claim 14 in the form of an injectable liquid solution, preferably for parenteral administration.

## Patentansprüche

1. Verfahren zur Herstellung von Adrenalin enthaltenden doppelschichtigen Lipidvesicula, bevozugt von Liposomen, umfassend die folgenden Schritte:
a) Herstellen einer Pufferlösung mit einem sauren pH zwischen 2 und 6 oder bevorzugt zwischen 2,4 und 3,0;
b) Zugeben einer Lipid enthaltenden Lösung zu der Pufferlösung und Halten unter Rühren, bis eine vesikuläre Dispersion gebildet ist;
c) Beschallen der Vesikeldispersion für eine Zeit zwischen 60 und 200 Sekunden oder bevorzugt zwischen 100 und 150 Sekunden;
d) Zugeben mindestens einer pharmazeutisch annehmbaren Antioxidanssubstanz;
e) Zugeben einer basischen Lösung zu der vesikulären Dispersion bis zum Erreichen eines pH-Werts von etwa 9, bevorzugt zwischen etwa 8 und 9, noch mehr bevorzugt zwischen etwa 8,7 und 9,0;
f) Zugeben von Adrenalin und Rühren der Dispersion für eine Zeit zwischen 5 und 15 Stunden, bevorzugt zwischen etwa 10 und 13 Stunden,
wobei die Schritte a)-f) in der beschriebenen Reihenfolge durchgeführt werden.

2. Verfahren nach Anspruch 1, wobei das Adrenalin in einer solchen Menge zugegeben wird, dass ein Lipid/Adrenalin-Gewichtsverhältnis von weniger als etwa 15, bevorzugt zwischen etwa 10 und 15, noch mehr bevorzugt gleich 14,6, erhalten wird.

3. Verfahren nach Ansprüchen 1-2, wobei die mindestens eine antioxidative Substanz ausgewählt ist aus Natriummetabisulfit, Kaliummetabisulfit, Natriumsulfit, Natriumthiosulfat, Methionin, Monothioglycerin, α-Tocopherol, Ascorbinsäure, Citronensäure, Butylhydroxyanisol (BHA), Butylhydroxytoluol (BHT) und Mischungen davon.

4. Adrenalinhaltige doppelschichtige Lipidvesikel zur Verwendung bei der Behandlung von Herznotfällen, die durch das Prozess nach einem der Ansprßche 1-3 erhaltbar sind, wobei die Doppelschicht im Wesentlichen umfasst:
i) ein oder mehrere gesättigte Phospholipide oder ein oder mehrere ungesättigte Phospholipide oder eine Mischung aus einem oder mehreren gesättigten Phospholipiden und einem oder mehreren ungesättigten Phospholipiden, und
ii) ein oder mehrere negativ geladene Lipide,
und wobei die Lipide, die die äußere Schicht der Lipiddoppelschicht bilden, an ein oder mehrere hydrophile Polymere gebunden sind, und die doppelschichtigen Lipidvesikel mindestens eine innere pharmazeutisch annehmbare antioxidative Substanz enthalten, wobei die Vesikel mehr als 75%, vorzugsweise etwa 90% des im Schritt f) addierten Adrenalins enthalten.

5. Lipidvesikel zur Verwendung nach Anspruch 4, wobei die Lipidvesikel Liposome sind.

6. Lipidvesikel zur Verwendung nach einem der Ansprüche 4-5, wobei das eine oder die mehreren Phospholipide ausgewählt sind aus der Gruppe bestehend aus : Dipalmitoylphosphatidylcholin (DPPC), Distearoylphosphatidylethanolamin (DSPE), Distearoylphosphatidylcholin (DSPC), Dipalmitoylphosphatidylethanolamin (DPPE), Dipalmitoylphosphorylglycerin (DPPG), Distearoylphosphorylglycerin (DSPG), Dipalmitoylphosphat (DPPA), Distearoylphosphat (DSPA), Dipalmitoylphosphatidylserin (DPPS), Distearoylphosphatidylserin (DSPS), Palmitoylstearoylphosphatidylcholin (PSPC), Dioleoylphosphatidylethanolamin (DOPE), Dioleoylphosphatidylcholin (DOPC), Palmitoyloleoylphosphatidylcholin (POPC), Stearoyl-oleoylphosphatidylcholin (SOPC); und Kombinationen davon.

7. Lipidvesikel zur Verwendung nach einem der Ansprüche 4-6, wobei das eine oder die mehreren negativ geladenen Lipide ausgewählt sind aus der Gruppe bestehend aus Phosphoglyceriden, Phosphatidylserin, Phosphatidylglycerin, Phosphatidylethanolamin, konjugiert an einen Liganden, und Kombinationen davon.

8. Lipidvesikel zur Verwendung nach einem der Ansprüche 4-7, wobei das eine oder die mehreren hydrophilen Polymere ausgewählt sind aus der Gruppe bestehend aus: Polyethylenglykol (PEG), Polyoxazolinen, Polyvinylalkoholen (PVA), Polyglycerin, Polyvinylpyrrolidon (PVP), Poly-N-hydroxypropylmethacrylamid (PolyHPMA), Polyaminosäuren und Kombinationen davon.

9. Lipidvesikel zur Verwendung nach Anspruch 8, wobei das hydrophile Polymer PEG ist.

10. Lipidvesikel zur Verwendung nach einem der Ansprüche 4-9, wobei die Doppelschicht ein Cholesterin umfasst.

11. Lipidvesikel zur Verwendung nach einem der Ansprüche 4-10, wobei die Vesikel einen mittleren Durchmesser zwischen 50 und 500 nm, mehr bevorzugt zwischen 50 und 200 nm, noch mehr bevorzugt zwischen 50 und 100 nm aufweisen.

12. Lipidvesikel zur Verwendung nach einem der Ansprüche 4-11, wobei di Vesikel unilamellar sind.

13. Lipidvesikel zur Verwendung nach einem der Ansprüche 4-12, wobei die Verwendung in der Behandlung von einem oder mehreren Zuständen liegt, die aus der Gruppe ausgewählt sind, die besteht aus: Herzstillstand, Kammerflimmern, pulsloser ventrikulärer Tachykardie, pulsloser elektrischer Herzaktivität, Herzasystolie und Kombinationen davon.

14. Pharmazeutische Zusammensetzung, umfassend die Adrenalin enthaltenden doppelschichtigen Lipidvesicula nach einem der Ansprüche 4-13.

15. Pharmazeutische Zusammensetzung zur Verwendung nach Anspruch 14 in Form als injizierbare flüssige Lösung, bevorzugt zur parenteralen Verabreichung.

## Revendications

1. Procédé de préparation de vésicules lipidiques à double couche contenant de l'adrénaline, comprenant les étapes consistant à :
a) préparer une solution tampon avec un pH acide compris entre environ 2 et 6, de préférence compris entre environ 2,4 et 3,0 ;
b) ajouter une solution contenant des lipides à la solution tampon et maintenir sous agitation jusqu'à la formation d'une dispersion vésiculaire;
c) soniquer la dispersion vésiculaire pendant un temps compris entre environ 60 et 200 secondes, ou de préférence entre environ 100 et 150 secondes;
d) ajouter au moins une substance anti-oxydante pharmaceutiquement acceptable;
e) ajouter une solution basique à la dispersion vésiculaire jusqu'à atteindre un pH d'environ 9, de préférence compris entre environ 8 et 9, encore plus de préférence compris entre environ 8,7 et 9,0;
f) ajouter de l'adrénaline et maintenir la dispersion sous agitation pendant un temps compris entre environ 5 et 15 heures, de préférence entre environ 10 et 13 heures ;
dans lequel les opérations a)-f) sont exécutées dans l'ordre décrit.

2. Procédé selon la revendication 1, dans lequel l'adrénaline est ajoutée en une quantité permettant d'obtenir un rapport pondéral lipides/adrénaline inférieur à environ 15, de préférence compris entre environ 10 et 15, encore plus de préférence égal à environ 14,6.

3. Procédé selon les revendications 1-2, dans lequel la au moins une substance anti-oxydante est choisie parmi le métabisulfite de sodium, le métabisulfite de potassium, le sulfite de sodium, le thiosulfate de sodium, la méthionine, le monothioglycérol, l'α-tocophérol, l'acide ascorbique, l'acide citrique, le butylhydroxyanisole (BHA), le butylhydroxytoluène (BHT) et leurs mélanges, de préférence le métabisulfite de sodium.

4. Vésicules lipidiques à double couche contenant de l'adrénaline destinée à être utilisée dans le traitement d'urgences cardiaques, qui peuvent être obtenues par le procédé selon une des revendications 1-3, dans lesquels la double couche comprend essentiellement:
i) un ou plusieurs phospholipides saturés, ou un ou plusieurs phospholipides insaturés, ou un mélange d'un ou plusieurs phospholipides saturés et d'un ou plusieurs phospholipides insaturés, et
ii) un ou plusieurs lipides chargés négativement,
et dans lesquels les lipides qui forment la couche externe de la double couche lipidique sont liés à un ou plusieurs polymères hydrophiles, et les vésicules lipidiques à double couche comprennent au moins une substance antioxidante pharmaceutiquement acceptable, les vésicules contenant plus de 75 %, de préférence plus de 90%, de l'adrénaline ajutée dans l'étape f).

5. Vésicules lipidiques pour l'utilisation dans la revendication 4, dans lesquelles les vésicules lipidiques sont des liposomes.

6. Vésicule lipidiques pour l'utilisation dans une des revendications 4-5, dans lesquelles les un ou plusieurs phospholipides sont choisis dans le groupe consistant en: la dipalmitoylphosphatidylcholine (DPPC), la distéaroylphosphatidyléthanolamine (DSPE), la distéaroylphosphatidylcholine (DSPC), la dipalmitoylphosphatidyléthanolamine (DPPE), le dipalmitoylphosphorylglycérol (DPPG), le distéaroylphosphorylglycérol (DSPG), le dipalmitoylphosphate (DPPA), le distéaroylphosphate (DSPA), la dipalmitoylphosphatidylsérine (DPPS), la distéaroylphosphatidylsérine (DSPS), la palmitoylstéaroylphosphatidylcholine (PSPC), la dioléoylphosphatidyléthanolamine (DOPE), la dioléoylphosphatidylcholine (DOPC), la palmitoyloléoylphosphatidylcholine (POPC), la stéaroyl-oléoylphosphatidylcholine (SOPC); et leur combinaisons.

7. Vésicules lipidiques pour l'utilisation dans une des revendications 4-6, dans lesquelles les un ou plusieurs lipides chargés négativement sont choisis dans le groupe consistant en phosphoglycérides, phosphatidylsérine, phosphatidylglycérol, phosphatidyléthanolamine conjugués à un ligand, et leurs combinaisons.

8. Vésicules lipidiques pour l'utilisation dans une des revendications 4-7, dans lesquelles les un ou plusieurs polymères hydrophiles sont choisis dans le groupe constitué de : polyéthylène glycol (PEG), polyoxazolines, alcools polyvinyliques (PVA), polyglycérol, polyvinylpyrrolidone (PVP), poly-N-hydroxypropylméthacrylamide (polyHPMA), polyaminoacides et leurs combinaisons.

9. Vésicules lipidiques pour l'utilisation selon la revendication 8, dans lesquelles le polymère hydrophile est le PEG.

10. Vésicules lipidiques pour l'utilisation dans une des revendications 4-9, dans lesquelles la double couche comprend un cholestérol.

11. Vésicules lipidiques pour l'utilisation dans une des revendications 4-10, dans lesquelles les vésicules ont un diamètre moyen compris entre 50 et 500 nm, de préférence compris entre 50 et 200 nm, et encore plus de préférence compris entre 50 et 100 nm.

12. Vésicules lipidiques pour l'utilisation dans une des revendications 4-11, dans lesquelles les vésicules sont unilamellaires.

13. Vésicules lipidiques pour l'utilisation dans une des revendications 4-12, dans lesquelles l'utilisation est dans le traitement d'une ou plusieurs conditions choisies dans le groupe consistant en : arrêt cardiaque, fibrillation ventriculaire, tachycardie ventriculaire sans pouls, activité électrique cardiaque sans pouls, asystole cardiaque et leurs combinaisons.

14. Composition pharmaceutique comprenant les vésicules lipidiques pour l'utilisation selon les revendications 4-13.

15. Composition pharmaceutique pour l'utilisation selon la revendication 14 sous forme d'une solution liquide injectable, de préférence pour une administration parentérale.
